# EUROPEAN PATENT SPECIFICATION

(11) **EP 3 891 153 B1**
(45) Date of publication and mention of the grant of the patent: **31.01.2024**
(21) Application number: 19817943.4
(22) Date of filing: 03.12.2019
(51) Int. Cl.: C07D 491/06, C09B 57/08

(54) **NAPHTHALIMIDOFURANS FOR APPLICATIONS IN MOLECULAR BIOLOGY**
NAPHTHALIMIDOFURANE FÜR ANWENDUNGEN IN DER MOLEKULARBIOLOGIE
NAPHTHALIMIDOFURANES DESTINÉS À ÊTRE UTILISÉS EN BIOLOGIE MOLÉCULAIRE

(30) Priority: 03.12.2018 CZ 20180671
(43) Date of publication of application: 13.10.2021
(73) Proprietor: Univerzita Karlova, 1. Lékarská Fakulta, 121 08 Praha 2 (CZ)
(72) Inventor: HAVLÍK, Martin, 16300 Praha 6 (CZ); TALIANOVÁ, Veronika, 97214 Tuzina (SK); BRÍZA, Tomás, 14800 Praha 4 (CZ); KAPLÁNEK, Robert, 10200 Praha 10 (CZ); MARTÁSEK, Pavel, 11917 Praha 9 (CZ); KRÁLOVÁ, Jarmila, 12000 Praha 2 (CZ); KRÁL, Vladimír, 12000 Praha 2 (CZ)
(74) Representative: Jirotkova, Ivana
(86) International application number: PCT/CZ2019/050060
(87) International publication number: WO 2020/114531

(56) References cited:
- EP-B1- 0 065 743
- WO-A1-95/00490
- MARTIN HAVLÍK ET AL: "Versatile fluorophores for bioimaging applications: [pi]-expanded naphthalimide derivatives with skeletal and appendage diversity", CHEMICAL COMMUNICATIONS, vol. 55, no. 18, 26 February 2019 (2019-02-26), pages 2696-2699, XP055662002, UK ISSN: 1359-7345, DOI: 10.1039/C8CC09638D
- XUHONG Q ET AL: "SYNTHESIS OF FURONAPHTHALIMIDES WITH POTENTIAL PHOTOSENSITIZING BIOLOGICAL ACTIVITY", DYES AND PIGMENTS, ELSEVIER APPLIED SCIENCE PUBLISHERS. BARKING, GB, vol. 25, no. 2, 1 January 1994 (1994-01-01), pages 109-114, XP000457119, ISSN: 0143-7208, DOI: 10.1016/0143-7208(94)85042-9 cited in the application
- BRANA MIGUEL F ET AL: "New analogues of amonafide and elinafide, containing aromatic heterocycles: Synthesis, antitumor activity, molecular modeling, and DNA binding properties", JOURNAL OF MEDICINAL CHEMISTRY, AMERICAN CHEMICAL SOCIETY, US, vol. 47, no. 6, 11 March 2004 (2004-03-11) , pages 1391-1399, XP002435350, ISSN: 0022-2623, DOI: 10.1021/JM0308850 cited in the application

## Description

### Technical Field

The invention concerns application of compounds based on naphthalimidofurans. These compounds can be used in the area of selective intracellular localization.

### Background Art

Spectroscopic methods based on the application of fluorescent probes are one of the most powerful and essential analytical technics, due to simple instrumental setup, low cost of their applications, high sensitivity, and their relative easy usability for *in vitro* and *in vivo* applications. [H. Kobayashi, M. Ogawa, R. Alford, P. L. Choyke and Y. Urano, New Strategies for Fluorescent Probe Design in Medical Diagnostic Imaging. Chem Rev, 2010, 110, 2620-2640]

The use of fluorescent probes is significantly involved this way significantly contributed to studding, understanding and explaining to number biochemical and biological phenomena and processes. In addition, suitable designed fluorescence probes and fluorophore labelled molecules (e.g. antibodies) are key parts of molecular imaging technics used in the modern diagnostic methods. [H. Kobayashi, M. Ogawa, R. Alford, P. L. Choyke and Y. Urano, New Strategies for Fluorescent Probe Design in Medical Diagnostic Imaging. Chem Rev, 2010, 110, 2620-2640] and optically controlled surgical operations. [G. A. Sonn, A. S. Behesnilian, Z. K. Jiang, K. A. Zettlitz, E. J. Lepin, L. A. Bentolila, S. M. Knowles, D. Lawrence, A. M. Wu and R. E. Reiter, Fluorescent Image-Guided Surgery with an Anti-Prostate Stem Cell Antigen (PSCA) Diabody Enables Targeted Resection of Mouse Prostate Cancer Xenografts in Real Time. Clin Cancer Res, 2016, 22, 1403-1412] Therefore, preparation and study of novel fluorophore structure motifs and development of new fluorescence probes are ones of the most important topic of analytical and bioanalytical chemistry.

There are several fluorophores commonly used in the biochemical and biological field. Among them, naphthalimide derivatives are one of the most studied fluorescent probes due to their excellent photophysical properties. Naphthalimide derivatives exhibit a wide range of suitable fluorescent properties such as high photostability, high fluorescence quantum yield, large Stokes shifts, photochemical stability and easy to modification of structure.

Naphthalimide derivatives are widely used in the construction of fluorescent sensors [R. M. Duke, E. B. Veale, F. M. Pfeffer, P. E. Kruger and T. Gunnlaugsson, Colorimetric and fluorescent anion sensors: an overview of recent developments in the use of 1,8-naphthalimide-based chemosensors. Chem Soc Rev, 2010, 39, 3936-3953] and cellular imaging agents. [S. Banerjee, E. B. Veale, C. M. Phelan, S. A. Murphy, G. M. Tocci, L. J. Gillespie, D. O. Frimannsson, J. M. Kelly and T. Gunnlaugsson, Recent advances in the development of 1,8-naphthalimide based DNA targeting binders, anticancer and fluorescent cellular imaging agents. Chem Soc Rev, 2013, 42, 1601-1618]

Coumarins (2H-1-benzopyran-2-one derivatives) as fluorophores are also known and studied for a long time. Together with relatively easy synthesis and variability of substitution pattern, coumarins are widely used as the fluorophore in the field of fluorescent chemosensors [K. Zamojc, M. Zdrowowicz, D. Jacewicz, D. Wyrzykowski and L. Chmurzynski, Fluorescent and Luminescent Probes for Monitoring Hydroxyl Radical under Biological Conditions. Crit Rev Anal Chem, 2016, 46, 160-169] as well as fluorescent dyes for labelling of biomolecules or cellular imaging. [Q. Zheng and L. D. Lavis, Development of photostable fluorophores for molecular imaging. Curr Opin Chem Biol, 2017, 39, 32-38; M. Tasior, D. Kim, S. Singha, M. Krzeszewski, K. H. Ahn and D. T. Gryko, pi-Expanded coumarins: synthesis, optical properties and applications. J. Mater. Chem. C, 2015, 3, 1421-1446]. Another type of used fluorophore is based on benzofuran skeleton. Similarly to coumarins and naphtalimides, benzo- and naphthofurans have suitable optical and physico-chemical properties for their utilisation as fluorophores for the construction of solvatochromic dyes [M. Koh, W. S. Kim and M. Lee, Exploration of a New Solvatochromic Dye Bearing the Excited-State Intramolecular Proton Transfer Functionality. Bull. Korean Chem. Soc., 2016, 37, 556-560], or fluorescent dyes for biological applications. [O. lamshanova, P. Mariot, V. Lehen'kyi and N. Prevarskaya, Comparison of fluorescence probes for intracellular sodium imaging in prostate cancer cell lines. Eur Biophys J, 2016, 45, 765-777; K. Nagy, E. Orban, S. Bosze and P. Kele, Clickable long-wave "mega-stokes" fluorophores for orthogonal chemoselective labeling of cells. Chem Asian J, 2010, 5, 773-777; G. Galvani, K. H. Vardhan Reddy, C. Beauvineau, N. Ghermani, F. Mahuteau-Betzer, M. Alami and S. Messaoudi, Conversion of 3-Bromo-2H-coumarins to 3-(Benzofuran-2-yl)-2H-coumarins under Palladium Catalysis: Synthesis and Photophysical Properties Study. Org. Lett., 2017, 19, 910-913]

In the development of novel fluorescent probes, there are two possible ways to make them: either combination of two or more fluorophores in one molecule (hybrid molecules, where each fluorophore is covalently linked to other) or preparation of fluorophore fused with other one or with (hetero)aromatic part, typically called as π-expanded fluorophores (preparation of novel fluorophore core), new entities.

Some naphthalimides with fused pyranone ring were mentioned in patent as compounds for dyeing of polyester fibers. [H. M. Deger, R. Erckel and H. Fruehbeis, Benzo[de]pyrano[3,2-g]isoquinoline derivatives useful as dyestuffs. Patent EP65743B1, 1982] They prepared one regioisomer of naphthalimide with fused pyranone ring by Knoevenagel condensation of 3-hydroxy-4-formyl-N-methylnaphthalimide with diethyl malonate or 2-(heteroaryl)acetonitrole yielded compounds with ethoxycarbonyl or (hetero)aromatic substitution on pyranone ring.

Naphthalimides with fused furan ring were already published as DNA intercalating and anticancer agents [C. Bailly, C. Carrasco, A. Joubert, C. Bal, N. Wattez, M. P. Hildebrand, A. Lansiaux, P. Colson, C. Houssier, M. Cacho, A. Ramos and M. F. Brana, Chromophore-Modified Bisnaphthalimides: DNA Recognition, Topoisomerase Inhibition, and Cytotoxic Properties of Two Mono- and Bisfuronaphthalimides. Biochemistry, 2003, 42, 4136-4150; M. F. Brana, M. Cacho, M. A. Garcia, B. de Pascual-Teresa, A. Ramos, M. T. Dominguez, J. M. Pozuelo, C. Abradelo, M. F. Rey-Stolle, M. Yuste, M. Banez-Coronel and J. C. Lacal, New Analogues of Amonafide and Elinafide, Containing Aromatic Heterocycles: Synthesis, Antitumor Activity, Molecular Modeling, and DNA Binding Properties. J. Med. Chem., 2004, 47, 1391-1399; Z. F. Tao, X. Qian, J. Tang, Synthesis of Furonaphthalimides as Novel DNA Intercalators. Dyes Pigments, 1996, 30, 247-252; M. Fernandez Brana, A. Ramos Gonzalez, M. Cacho Izquierdo, S. Rajamaki, N. Acero de Mesa, J. M. Pozuelo Gonzalez, D. Munoz-Mingarro Martinez, Preparation of 1,8-naphthalimide derivatives for therapeutic use as antiproliferative /anticancer agents, Patent ES2191532B1, 2003; A. D. Patten, G. J. Pacofsky, S. P. Seitz, E. A. Akamike, R. J. Cherney, R. F. Kaltenbach, M. J. Orwat, Preparation of bis-imide polycyclic and heterocyclic chromophores as tumoricidals. WO9500490A1, 1995 (Taiwan and JAR); X. Qian, H. Yin, Y. Xu, M. Xiao, J. Li, L.Lin, J. Ding, Preparation of naphthalimide derivatives for inhibiting growth of tumor cell and mutant cell. CN100465177C, 2007], photosensitizers [X. H. Qian, J. Tang, J. D. Zhang and Y. L. Zhang, Synthesis of Furonaphthalimides with Potential Photosensitizing Biological Activity. Dyes Pigments, 1994, 25, 109-114], compounds for studies of fluorescent properties [X. Qian, Y. Zhang, K. Chen, Z. Tao, Y. Shen, A study on the relationship between Stoke's shift and low frequency half-value component of fluorescent compounds. Dyes Pigments, 1996, 32, 229-235] and chemiluminescent probes for singlet oxygen [W. Adam, X. H. Qian and C. R. Sahamoller, Synthesis and Photooxygenation of 2,3,6-Trimethylfuro[2,3-b] [I] naphtho[4a, 7a-e,f)pyrida-5,7-dione, A Potential Chemiluminescent Probe for Singlet Oxygen. Tetrahedron, 1993, 49, 417-422] Unfortunately, described syntheses yielded derivatives without any group on furan ring suitable for further synthetic modification, that is absolutely crucial for their use in molecular biology (the derivatives prepared did not have a furan ring substituted or carried methyl groups).

We prepared two regioisomers of naphthalimidofuran by Rap-Stoermer reaction of naphthalimido-o-hydroxycarbaldehydes with ethyl bromoacetate in the presence of a base. In addition, for comparison of properties, we prepared two regioisomers of naphthalimidopyranone by reaction of naphthalimide-o-hydroxycarbaldehydes with ethyl malonate in the presence of piperidine. Both synthetic methods provided derivatives with ethoxycarbonyl group on new ring, which is suitable for further synthetic modifications.

### Summary of Invention

The subject hereof are naphthalimidofurans with functional groups and their application as fluorescent probes.

These fluorescent probes possess two appendages (functional groups suitable for further modification): one on imide nitrogen (R1) and the other on furan ring (R2). Suitable groups on these positions can serve as targeting groups for specific and selective subcellular localisation, reactive site for connection to biomolecules or as a modulator of solubility and lipophilicity.

We have prepared fluorescent probes for lysosomal imaging bearing alkyl group on imide nitrogen for the fast transport to cell and basic N-(2-(dimethylamino)ethyl)amide group for lysosomal targeting on pyranone or furan ring. Lysosomal probes based on naphthalimidofurans showed better photophysical and photochemical properties than the corresponding naphthalimidopyranones.

The subject of invention are naphthalimidofurans with functional groups of general formula I and their application as fluorescent probes. Fluorescent probes show highly selective lysosomal localisation and they also show excellent brightness, high photostability and low toxicity.

The subject of the invention are naphthalimidofurans of general formula I: 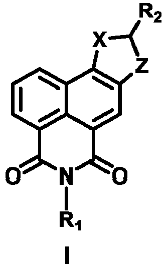
where R₁ is H, alkyl C1 to C12, allyl, propargyl, benzyl, glycol chains with 1 to 8 glycol (-OCH₂CH₂-) repeating units terminated with O-alkyl substituent C1 to C12 or OH group,
where R₂ is COOR₁, CON(R₁)₂, where both R1 substituents may be the same or different, CONH(CH₂)ₓN(R₁)₂, where x is 2 to 6
and wherein both R1 substituents may be the same or different, CONH(CH₂)ₓNY, where x is as defined above and Y is -CH₂-A-CH₂CH₂-, where A is CH₂, CH₂CH₂, CH₂O, CH₂S, CH₂SO, CH₂SO₂, CH₂NH, CH₂NR₁,
where X = O, Z = CH; R₂C and Z are linked by a double bond (R2C=Z) or where X = CH, Z = O; R₂C and X are linked by a double bond (R2C=X).

Compounds of general formula I have fluorescent properties; R1 and R2 substituents are functional groups providing selective localization to cellular organelles, aiding solubility and affecting lipophilicity, therefore, their great variability is possible. The compounds of general formula I can be used in the field of selective intracellular localization as a fluorescent probe for the selective labeling of cellular organelles. These substances are relatively easy to prepare, have low toxicity and exhibit the desired properties to find use in fluorescence microscopy for biological studies as fluorescent probes.

The preparation and properties of naphthalimidofurans and their application as fluorescent probes are documented bellow.

### Brief Description of Drawings

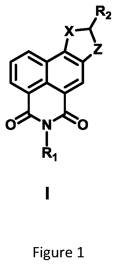

- Fig. 1: Structural formula of naphthalimidofurans
- Fig. 2: Fluorescence spectra of fluorescent probes 2 (LysoSer **2,** for reference only), **4** (LysoSer **4,** for reference only), 6 (LysoSer 6), 8 (LysoSer 8) and 10 (LysoSer 10) in four different solvents (PBS buffer, ethanol, methanol, DMSO).
- Fig. 3: Fluorescence spectra of fluorescent probes 2, 4, 6, 8 (LysoSer 16) in four different solvents (PBS buffer, ethanol, methanol, DMSO). Probes 2 and 4 are for reference only.
- Fig. 4: Fluorescence and intracellular localization of fluorescent probes 2, 4 (1µM, for reference only), probes 6, 8 (200
nM) for the lysosomal targeting. (A) Images of the same field of cells showing phase contrast (left column) and cellular fluorescence (right column). (B) Lysosomal localization of probes. Fluorescent probes were co-labeled by the commercial specific lysosomal probe LysoTracker Green DND-26 (LT-G) in three cell line (HF-P4, BLM, a A-2058). Images were captured with Leica TCS SP8 WLL SMD-FLIM confocal microscope using an HC PL APO CS2 63x / 1.2W watertight lens.

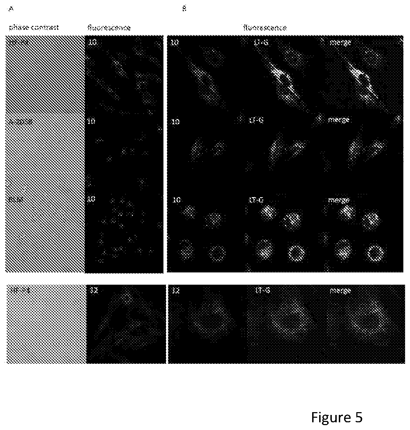

- Fig. 5: Fluorescence and intracellular localization of fluorescent probe 10 (top - 50 nM, middle - 100 nM, bottom - 200 nm), probe **12** (200 nM) for the lysosomal targeting. (A) Images of the same field of cells showing phase contrast (left column) and cellular fluorescence (right column). (B) Lysosomal localization of probes Fluorescent probes were co-labeled by the commercial specific lysosomal probe LysoTracker Green DND-26 (LT-G) in three-cell line (HF-P4, BLM, a A-2058). Images were captured with Leica TCS SP8 WLL SMD-FLIM confocal microscope using an HC PL APO CS2 63x / 1.2W watertight lens.

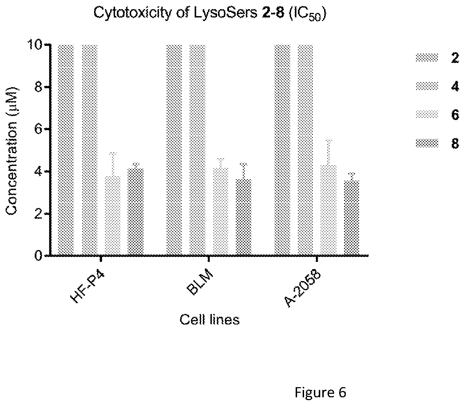

- Fig. 6: Cytotoxicity assay of fluorescent probes **2** (LysoSer 13, for reference only), **4** (LysoSer 14, for reference only), **6** (LysoSer 15) and **8** (LysoSer 16) in three-cell line (HF-P4, BLM, a A-2058).

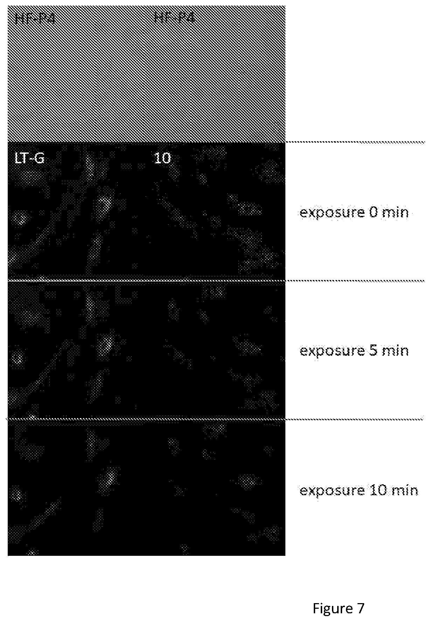

- Fig.7: Photobleaching of fluorescent probe **10** (LysoSers 10) and LysoTracker Green DND-26 (LT-G).

### Examples

### Example 1 (for reference only)

### Preparation of fluorescent probe 1

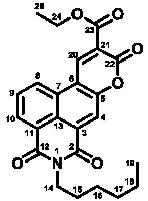

2-Hexyl-5-hydroxy-1,3-dioxo-2,3-dihydro-1*H*-benzo[*de*]isochinolin-6-carbaldehyde (650 mg, 2,0 mmol) Aldehyde 3 (650 mg, 2.0 mmol) was treated with diethyl malonate (0.4 mL, 420 mg, 2.6 mmol) and piperidine (5 drops) in ethanol (15 mL) at reflux temperature overnight. The reaction mixture was evaporated to dryness *in vacuo.* The residue was purified by flash chromatography (dichloromethane/methanol 97:3) to give 620 mg (74%) of probe 1. ¹H NMR (500 MHz, CDCl₃): 9.28 (1H, d, 0.8, H20), 8.67 (1H, dd, 7.3, 1.0, H10), 8.64 (1H, dd, 8.5, 1.0, H8), 8.52 (1H, d, 0.8, H4), 8.00 (1H, dd, 8.5, 7.3, H9), 4.50 (2H, q, 7.1, H24), 4.18 (2H, ^{~}t, 7.7, H14), 1.73 (2H, ^{~}kv, 7.7, H15), 1.47 (3H, t, 7.1, H25), 1.43 (2H, m, H16), 1.38-1.29 (4H, m, H17 and H18), 0.89 (3H, ^{~}t, 7.2, H19). ¹³C NMR (126 MHz, CDCl₃): 163.40 (C12), 163.10 (C23), 162.58 (C2), 155.66 (C22), 155.34 (C5), 142.79 (C20), 130.83 (C10), 129.80 (C9), 128.74 (C7), 128.19 (C3), 127.43 (C8), 125.23 (C13), 123.83 (C11), 121.07 (C4), 120.61 (C21), 116.46 (C6), 62.79 (C24), 41.15 (C14), 31.64 (C17), 28.14 (C15), 26.90 (C16), 22.69 (C18), 14.41 (C25), 14.17 (C19). HRMS (ESI⁺): calcd. for C₂₄H₂₃N₁O₆Na₁ [M + Na]⁺ 444.14176; found 444.14185.

### Example 2 (for reference only)

### Preparation of fluorescent probe 2

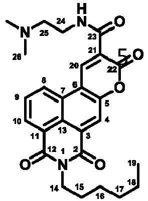

Probe 1 (100 mg, 0.24 mmol) was treated with 2M aqua solution of sodium hydroxide (5 mL) in ethanol (5 mL) at reflux temperature for 2h. Acetic acid was added until pH of solution was not slightly acidic. Formed solid was filtered off, washed by water and dried *in vacuo* to obtain 90 mg (96%) of corresponding acid. Acid was used to next step without next purification. Acid (85 mg, 0.22 mmol) was treated with 2M solution of oxalyl chloride (3 mL) in dichloromethane with *N*,*N*-dimethylformamide (2 drops) as catalyst in dichloromethane (5 mL) at room temperature for 2h. The reaction mixture was evaporated to dryness *in vacuo* to obtain 89 mg (100%) of corresponding chloride of acid. Prepared chloride acid (89 mg, 0.22 mmol) was treated with *N*,*N*-dimethylethylenediamine (25 mg, 0.28 mmol) and with triethylamine (36 mg, 0.36 mmol) in dichloromethane (5 mL) at room temperature overnight. The reaction mixture was evaporated to dryness *in vacuo* and the residue was purified by flash chromatography (dichloromethane/methanol 9:1) to give 88 mg (88%, 84% overall yield) of probe 2. ¹H NMR (500 MHz, CDCl₃): 9.61 (1H, s, H20), 9.19 (1H, br t, 5.9, NH), 8.73 (1H, dd, 8.4, 1.0, H8), 8.67 (1H, dd, 7.4, 1.0, H10), 8.51 (1H, d, 0.6, H4), 8.00 (1H, dd, 8.4, 7.4, H9), 4.17 (2H, ^{~}t, 7.7, H14), 4.02 (2H, br q, 5.9, H24), 3.30 (2H, br s, H25), 2.89 (6H, br s, H26), 1.73 (2H, ^{~}kv, 7.6, H15), 1.43 (2H, ^{~}kv, 7.5, H16), 1.39-1.29 (4H, m, H17 and H18), 0.90 (3H, t, 7.1, H19). ¹³C APT NMR (126 MHz, CDCl₃): 163.33 (C12), 162.51 (C2 or C23), 162.45 (C2 or C23), 160.22 (C22), 154.46 (C5), 142.68 (C20), 131.06 (C10), 129.91 (C9), 128.82 (C7), 128.14 (C3), 127.93 (C8), 125.35 (C13), 123.71 (C11), 120.75 (C4), 120.33 (C21), 117.28 (C6), 56.89 (C25), 43.88 (C26), 41.16 (C14), 35.51 (br, C24), 31.64 (C17), 28.13 (C15), 26.90 (C16), 22.70 (C18), 14.18 (C19). HRMS (ESI⁺): calcd. for C₂₆H₃₀N₃O₅ [M + H]⁺ 464.21800; found 464.21829.

### Example 3 (for reference only)

### Preparation of fluorescent probe 3

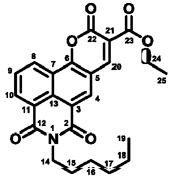

2-Hexyl-6-hydroxy-1,3-dioxo-2,3-dihydro-1*H*-benzo[de]isoquinolin-5-karbaldehyd (650 mg, 2.0 mmol) was treated with diethyl malonate (0.4 mL, 420 mg, 2.6 mmol) and piperidine (5 drops) in ethanol (15 mL) at reflux temperature overnight. The reaction mixture was evaporated to dryness *in vacuo.* The residue was purified by flash chromatography (dichloromethane/methanol 97:3) to give 590 mg (70%) of probe 3. ¹H NMR (500 MHz, CDCl₃): 8.87 (1H, dd, 8.4, 1.2, H8), 8.77 (1H, dd, 7.4, 1.2, H10), 8.76 (1H, s, H20), 8.71 (1H, s, H4), 7.94 (1H, dd, 8.4, 7.4, H9), 4.47 (2H, q, 7.2, H24), 4.17 (2H, ^{~}t, 7.7, H14), 1.73 (2H, m, H15), 1.45 (3H, t, 7.2, H25), 1.45-1.30 (6H, m, H16, H17 and H18), 0.89 (3H, ^{~}t, 7.1, H19). ¹³C NMR (126 MHz, CDCl₃): 163.56 (C12), 162.90 (C2), 162.54 (C23), 156.44 (C6), 155.35 (C22), 148.78 (C20), 134.18 (C10), 130.89 (C4), 130.45 (C13), 129.12 (C8), 128.57 (C9), 123.26 (C11), 121.31 (C7), 119.87 (C3), 119.08 (C21), 114.26 (C5), 62.55 (C24), 40.92 (C14), 31.66 (C17), 28.18 (C15), 26.91 (C16), 22.70 (C18), 14.38 (C25), 14.18 (C19). HRMS (ESI⁺): calcd. for C₂₄H₂₃N₁O₆Na [M + Na]⁺ 444.14176; found 444.14184.

### Example 4 (for reference only)

### Preparation of fluorescent probe 4

Probe 3 (130 mg, 0.30 mmol) was treated with 2M aqua solution of sodium hydroxide (5 mL) 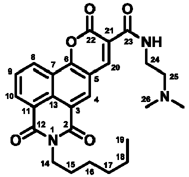 in ethanol (5 mL) at reflux temperature for 2h. Acetic acid was added until pH of solution was not slightly acidic. Formed solid was filtered off, washed by water and dried *in vacuo* to obtain 115 mg (95%) of corresponding acid. Acid was used to next step without next purification. Acid (110 mg, 0.28 mmol) was treated with 2M solution of oxalyl chloride (3 mL) in dichloromethane with *N*,*N*-dimethylformamide (2 drops) as catalyst in dichloromethane (5 mL) at room temperature for 2h. The reaction mixture was evaporated to dryness *in vacuo* to obtain 115 mg (100%) of corresponding chloride of acid. Prepared chloride acid (115 mg, 0.28 mmol) was treated with *N*,*N*-dimethylethylenediamine (27 mg, 0.31 mmol) and with triethylamine (40 mg, 0.40 mmol) in dichloromethane (5 mL) at room temperature overnight. The reaction mixture was evaporated to dryness *in vacuo* and the residue was purified by flash chromatography (dichloromethane/methanol 9:1) to give 110 mg (85%, 81% overall yield) of probe 4. ¹H NMR (500 MHz, CDCl₃): 9.11 (1H, d, 0.4, H20), 8.93 (1H, br t, 5.3, NH), 8.84 (1H, dd, 8.4, 1.2, H8), 8.77 (1H, dd, 0.4, 0.3, H4), 8.76 (1H, dd, 7.4, 1.2, H10), 7.94 (1H, ddd, 8.4, 7.4, 0.3, H9), 4.18 (2H, ~t, 7.7, H14), 3.69 (2H, q, 5.9, H24), 2.72 (2H, br t, 6.5, H25), 2.44 (6H, s, H26), 1.73 (2H, ~kv, 7.6, H15), 1.43 (2H, ~kv, 7.5, H16), 1.38-1.27 (2H, m, H17 and H18), 0.89 (3H, t, 7.2, H19). ¹³C NMR (126 MHz, CDCl₃): 163.52 (C12), 162.83 (C2), 161.18 (C23), 160.06 (C22), 155.28 (C6), 148.38 (C20), 133.99 (C10), 131.14 (C4), 130.26 (C13), 128.77 (C8), 128.62 (C9), 123.31 (C11), 121.21 (C7), 120.24 (C3), 119.65 (C21), 115.18 (C5), 57.75 (C25), 45.15 (C26), 40.94 (C14), 37.51 (C24), 31.66 (C17), 28.17 (C15), 26.90 (C16), 22.69 (C18), 14.18 (C19). HRMS (ESI⁺): calcd. for C₂₆H₃₀N₃O₅ [M + H]⁺ 464.21800; found 464.21837.

### Example 5

### Preparation of fluorescent probe 5, with general formula I

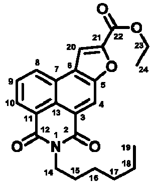

2-Hexyl-5-hydroxy-1,3-dioxo-2,3-dihydro-1*H*-benzo[*de*]isoquinolin-6-carbaldehyde (650 mg, 2.0 mmol) was treated with ethyl bromoacetate (0.3 mL, 453 mg, 2.7 mmol) and potassium carbonate (830 mg, 6.0 mmol) in acetonitrile (15 mL) at reflux temperature for 2 days. The reaction mixture was evaporated to dryness *in vacuo.* The residue was purified by flash chromatography (dichloromethane) to give 450 mg (57%) of probe 5. ¹H NMR (500 MHz, CDCl₃): 8.83 (1H, d, 1.0, H4), 8.65 (1H, dd, 7.4, 1.2, H10), 8.49 (1H, dd, 8.2, 1.2, H8), 8.07 (1H, d, 1.0, H20), 7.90 (1H, dd, 8.2, 7.4, H9), 4.53 (2H, q, 7.1, H23), 4.20 (2H, ^{~}t, 7.7, H14), 1.75 (2H, ^{~}kv, 7.6, H15), 1.49 (3H, t, 7.1, H24), 1.44 (2H, ^{~}kv, 7.5, H16), 1.36 (2H, m, H17), 1.35 (2H, m, H18), 0.90 (3H, t, 7.1, H19). ¹³C NMR (126 MHz, CDCl₃): 164.10 (C12), 163.88 (C2), 158.88 (C22), 153.43 (C5), 148.82 (C21), 130.06 (C10), 129.34 (C8), 128.14 (C9), 128.07 (C6), 126.71 (C7), 125.71 (C13), 123.83 (C11), 122.92 (C3), 117.97 (C4), 112.27 (C20), 62.30 (C23), 41.00 (C14), 31.70 (C17), 28.22 (C15), 26.96 (C16), 22.72 (C18), 14.49 (C24), 14.20 (C19). HRMS (ESI⁺): calcd. for C₂₃H₂₄N₁O₅ [M + H]⁺ 394.16490; found 394.16513.

### Example 6

### Preparation of fluorescent probe 6, with general formula I

Probe 5 (118 mg, 0.3 mmol) was treated with *N*,*N*-dimethylethylenediamine (1 mL) in ethanol 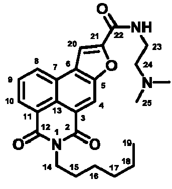 (10 mL) at reflux temperature overnight. The reaction mixture was evaporated to dryness *in vacuo.* The residue was purified by flash chromatography (dichloromethane/methanol 9:1) to obtain 112 mg (86%) of probe 6. ¹H NMR (500 MHz, CD₃SOCD₃): 8.86 (1H, dd, 8.2, 1.2, H8), 8.85 (1H, br t, 5.8, NH), 8.67 (1H, d, 1.0, H4), 8.53 (1H, dd, 7.4, 1.2, H10), 8.39 (1H, d, 1.0, H20), 8.00 (1H, ddd, 8.2, 7.4, 0.3, H9), 4.06 (1H, ~t, 7.5, H14), 3.45 (2H, td, 6.7, 5.8, H23), 2.47 (2H, covered, H24), 2.24 (6H, s, H25), 1.65 (1H, ~kv, 7.4, H15), 1.36 (2H, m, H16), 1.35-1.26 (4H, m, H17 and H18), 0.87 (3H, ~t, 7.1, H19). ¹³C NMR (126 MHz, CD₃SOCD₃): 163.30 (C12), 163.12 (C2), 157.37 (C22), 152.23 (C21), 151.45 (C5), 130.26 (C8), 129.37 (C10), 128.56 (C6), 128.08 (C9), 126.13 (C7), 124.78 (C13), 122.81 (C11), 121.14 (C3), 116.58 (C4), 109.13 (C20), 57.84 (C24), 45.07 (C25), 39.94 (C14), 36.86 (C23), 30.93 (C17), 27.39 (C15), 26.17 (C16), 21.94 (C18), 13.89 (C19). HRMS (ESI⁺): calcd. for C₂₅H₃₀N₃O₄ [M + H]⁺ 436.22308; found 436.22327.

### Example 7

### Preparation of fluorescent probe 7, with general formula I

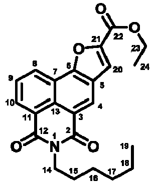

2-hexyl-6-hydroxy-1,3-dioxo-2,3-dihydro-1H-benzo[de]isoquinolin-5-carbaldehyde (650 mg, 2.0 mmol) was treated with ethyl bromoacetate (0.3 mL, 453 mg, 2.7 mmol) and potassium carbonate (830 mg, 6.0 mmol) in acetonitrile (15 mL) at reflux temperature for 2 days. The reaction mixture was evaporated to dryness *in vacuo.* The residue was purified by flash chromatography (dichloromethane) to give 320 mg (41%) of probe 7. ¹H NMR (500 MHz, CDCl₃): 8.87 (1H, s, H4), 8.76 (1H, dd, 8.2, 1.2, H8), 8.66 (1H, dd, 7.4, 1.2, H10), 7.89 (1H, ddd, 8.2, 7.4, 0.3 to H4, H9), 7.78 (1H, s, H20), 4.50 (2H, q, 7.1, H23), 4.18 (2H, ^{~}t, 7.7, H14), 1.74 (2H, ^{~}kv, 7.7, H15), 1.47 (3H, t, 7.1, H24), 1.44 (2H, ^{~}kv, 7.4, H16), 1.38-1.29 (4H, m, H17 and H18), 0.89 (3H, ^{~}t, 7.2, H19). ¹³C NMR (126 MHz, CDCl₃): 164.18 (C12), 163.95 (C2), 158.89 (C22), 154.59 (C6), 147.25 (C21), 131.08 (C10), 127.82 (C9), 127.78 (C13), 127.47 (C4), 126.99 (C8), 123.86 (C5), 123.51 (C11), 119.92 (C3), 119.54 (C7), 115.21 (C20), 62.07 (C23), 40.86 (C14), 31.70 (C17), 28.22 (C15), 26.95 (C16), 22.71 (C18), 14.49 (C19), 14.19 (C24). HRMS (ESI⁺): calcd. for C₂₃H₂₄N₁O₅ [M + H]⁺ 394.16490; found 394.16459.

### Example 8

### Preparation of fluorescent probe 8, with general formula I

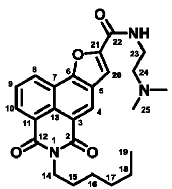

Probe **7** (118 mg, 0.3 mmol) was treated with *N,N-*dimethylethylenediamine (1 mL) in ethanol (10 mL) at reflux temperature overnight. The reaction mixture was evaporated to dryness *in vacuo.* The residue was purified by flash chromatography (dichloromethane/methanol 9:1) to obtain 108 mg (83%) of probe **8.** ¹H NMR (500 MHz, CD₃SOCD₃): 8.88 (1H, br t, 5.7, NH), 8.86 (1H, s, H4), 8.77 (1H, dd, 8.2, 1.2, H8), 8.53 (1H, dd, 7.4, 1.2, H10), 8.02 (1H, dd, 8.2, 7.4, H9), 7.86 (1H, s, H20), 4.04 (2H, ^{~}t, 7.5, H14), 3.46 (2H, td, 6.7, 5.7, H23), 2.52 (2H, t, 6.7, H24), 2.25 (6H, s, H25), 1.63 (2H, ^{~}kv, 7.4, H15), 1.35 (2H, m, H16), 1.36-1.25 (4H, m, H17 and H18), 0.86 (3H, ~t, 7.1, H19). ¹³C NMR (126 MHz, CD₃SOCD₃): 163.35 (C2), 163.10 (C12), 157.40 (C), 152.50 (C6), 150.26 (C21), 130.03 (C10), 127.97 (C9), 127.31 (C4), 126.45 (C13), 126.35 (C8), 124.11 (C5), 122.79 (C11), 118.76 (C3), 118.63 (C7), 111.08 (C20), 58.01 (C24), 45.09 (C25), 39.80 (C14), 36.82 (C23), 30.94 (C17), 27.41 (C15), 26.17 (C16), 21.96 (C18), 13.89 (C19). HRMS (ESI⁺): calcd. for C₂₅H₃₀N₃O₄ [M + H]⁺ 436.22308; found 436.22343.

### Example 9

### Preparation of fluorescent probe 9, with general formula I

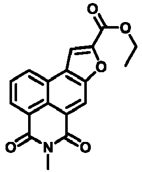

Probe **9** was prepared as in Example 5, using 2-methyl-5-hydroxy-1,3-dioxo-2,3-dihydro-1H-benzo[de]isoquinolin-6-carbaldehyde (510 mg, 2,0 mmol) as starting compound to obtain330 mg (51%) of probe **9.**

### Example 10

### Preparation of fluorescent probe 10, with general formula I

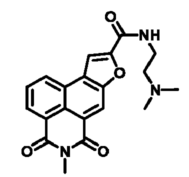

Probe **10** was prepared as in Example 6, using probe **9** (65 mg, 0,2mmol) as starting compound to obtain 65 mg (88%) of probe **10.**

### Example 11

### Preparation of fluorescent probe 11, with general formula I

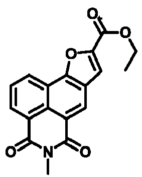

Probe **11** was prepared as in Example 7, using 2-methyl-6-hydroxy-1,3-dioxo-2,3-dihydro-1H-benzo[de]isoquinolin-5-carbaldehyde (510 mg, 2 mmol) as starting compound to obtain 300 mg (46%) of probe **11.**

### Example 12

### Preparation of fluorescent probe 12, with general formula I

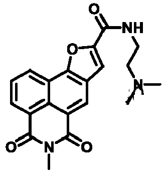

Probe **12** was prepared as in Example 8, using probe **11** (65 mg, 0,2mmol) as starting compound to obtain 60 mg (82%) of probe **12.**

### Example 13

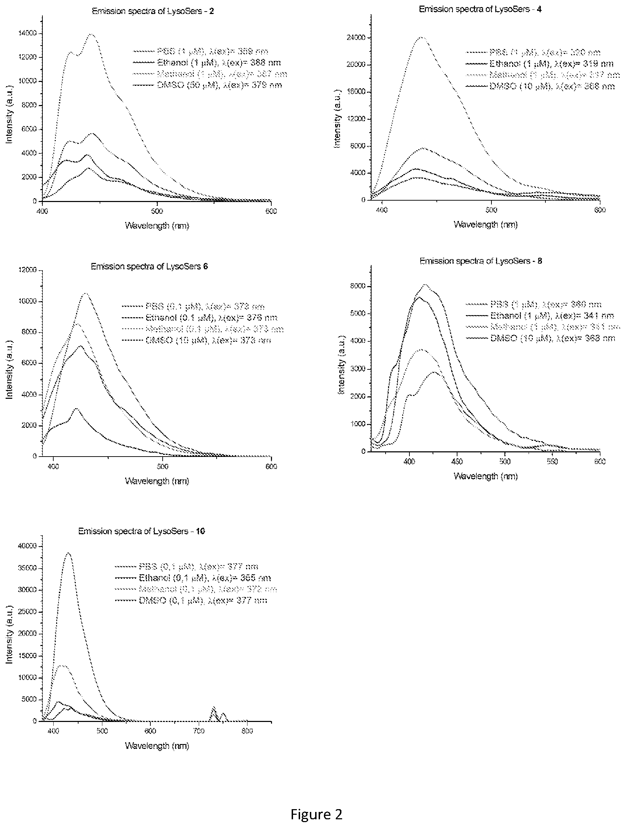

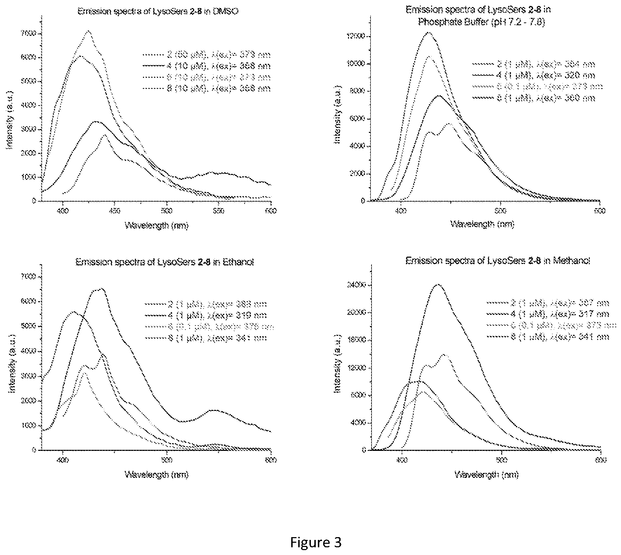

Spectral properties of fluorescent probes **2, 4, 6, 8** and **10 (2** and **4** are for reference only) Spectral characteristics of probes were obtained by using a UV/VIS and fluorescence studies. The probes were measured in four solvents (DMSO, methanol, ethanol and phosphate buffer). Each LysoSers was at first dissolved in DMSO 1mM stock solution which was diluted to a final concentration 1 µM or 0,1 µM. Obtained spectra and values of absorbance and emission maxima of the individual fluorescent probes are shown in Table 1 and Figure 2 and 3.

All prepared probes have absorption maximum in the ultraviolet range of 350-400 nm and emission maximum in the range of 400 to 450 nm. Probes **6** (LysoSer 6), **8** (LysoSer 8) and **10** (Lysoser 10) with furan motive showed higher fluorescence intensities than probes **2** (LysoSer 2) and **4** (LysoSer 4) with pyranone motive.

**Table 1. Absorption and emission maxima of probes 2, 4, 6, 8 and 10 in different solvents (probes 2 and 4 are for reference only)**

| **Probe** | **Solvent** | **Absorbance maxima (nm)** | **Emission maxima (nm)** |
|---|---|---|---|
| **2** | DMSO | 379 | 440 |
| | EtOH | 388 | 448 |
| | MeOH | 387 | 442 |
| | PBS | 389 | 448 |
| **4** | DMSO | 368 | 432 |
| | EtOH | 319 | 434 |
| | MeOH | 317 | 435 |
| | PBS | 320 | 437 |
| **6** | DMSO | 373 | 424 |
| | EtOH | 376 | 422 |
| | MeOH | 373 | 421 |
| | PBS | 373 | 429 |
| **8** | DMSO | 368 | 416 |
| | EtOH | 341 | 415 |
| | MeOH | 341 | 416 |
| | PBS | 360 | 428 |
| **10** | DMSO | 377 | 435 |
| | EtOH | 365 | 410 |
| | MeOH | 372 | 420 |
| | PBS | 377 | 431 |

### Example 14

### Intracellular studies of fluorescent probes

Intracellular localization of the fluorescent probes **2, 4, 6, 8, 10** and **12 (2** and **4** are for reference only) in living cells was studied on a confocal microscope Leica TCS SP8 WLL SMD-FLIM (equipped with 405 nm pulsed laser) at 37 ° C and 0.05 tension of CO₂ using an HC PL APO CS2 63x / 1.2W watertight lens. The ability of efficiency of probes have enter and eventually stained in specific intracellular compartments (lysosomes) was tested using three different cell lines (HF-P4 - human fibroblast cells, and two melanoma cell lines A-2058 and BLM). As the co-localizing agent was used LysoTracker Green DND-26 (LT-G). Cells were simultaneously stained with probes **2, 4** (1 µM), probes **6, 8** (200 nM), probe **10** (50 nM), probe **12** (200 nM) and LysoTracker Green DND-26 (200 nM) for 30 minutes under growth conditions and images were captured by a confocal microscope.

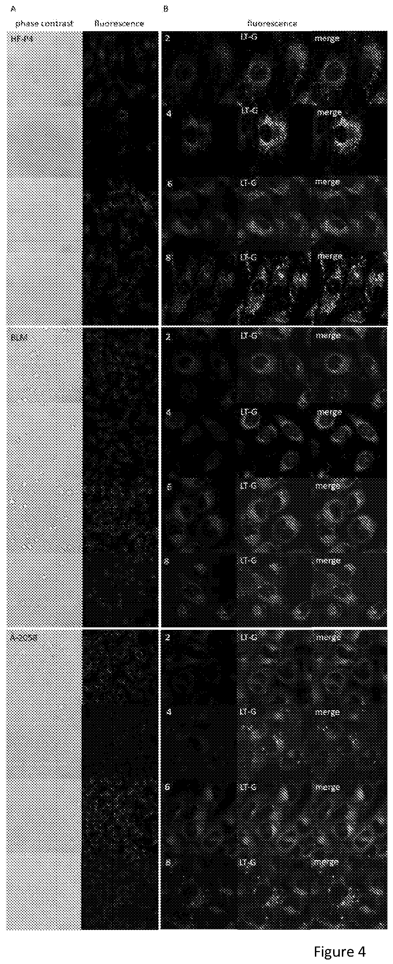

The measurements confirmed that all probes stain the lysosomal compartment similarly to LysoTracker Green DND-26 (Figure 4-5). All probes provided blue intensive fluorescence. Fluorescent probes with furan ring have significantly better brightness than corresponding probes with pyranone ring

### Example 15

### Cytotoxicity assay of fluorescent probes

A colorimetric cell metabolic activity assay was used for the cytotoxicity test of the fluorescent probes **2, 4, 6** and **8 (2** and **4** are for reference only).

Cells were cultured under standard conditions in a culture medium (DMEM with 10% FBS). After 24 hours of cultivation, cells were plated on 96-well plates at a density of 5000 cells per well. The cells were stored under standard culture conditions for next 24h. After this time, the medium was removed and medium with tested compounds was added and cultivated for 48h. Afterwards, the medium was exchanged for tetrazolium dye MTT (3-(4,5-dimethylthiazol-2-yl)-2,5-diphenyltetrazolium bromide, purchased from Sigma-Aldrich) and incubated for 2h (37°C). After a double hour incubation, the yellow tetrazole dye was removed and DMSO was added, which reduced to purple formazan in living cells. This was followed by testing with the (NanoQuant microplate reader by Tecan) An absorbance of converted dye was measured at a wavelength of 570 nm with the reference of 630 nm. Based on this test the value - IC₅₀ was determined (concentration inhibiting the growth of cells). Measurements of cytotoxicity of fluorescent probes at various concentrations (0-10 µM) showed almost no or low toxicity (Figure 6). These concentrations are one to two orders of magnitude higher than those used to measure their intracellular localization.

### Example 16

Photostability of prepared probes is shown as a photobleaching in HF-P4 cell line (Figure 7).

Figure shows bleaching of probe **10** and LysoTracker Green DND-26 (LT-G).

## Claims

1. Naphthalimidofurans of general formula I: 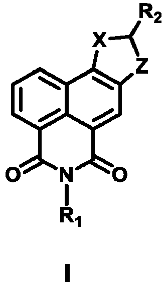
where R₁ is H, alkyl C1 to C12, allyl, propargyl, benzyl, glycol chains with 1 to 8 glycol (-OCH₂CH₂-) repeating units terminated with O-alkyl substituent C1 to C12 or OH group,
where R₂ is COOR₁, CON(R₁)₂, where both R₁ substituents may be the same or different, CONH(CH₂)ₓN(R₁)₂, where x is 2 to 6 and wherein both R1 substituents may be the same or different, CONH(CH₂)ₓNY, where x is as defined above and Y is -CH₂-A-CH₂CH₂-, where A is CH₂, CH₂CH₂, CH₂O, CH₂S, CH₂SO, CH₂SO₂, CH₂NH, CH₂NR₁,
where X = O, Z = CH; R₂C and Z are linked by a double bond (R₂C=Z) or where X = CH, Z = O; R₂C and X are linked by a double bond (R₂C=X).

2. Use of naphthalimidofurans of general formula I according to claim 1 for preparation of fluorescence probes for selective intracellular localisation.

3. Use of naphthalimidofurans of general formula I according to claim 1 as fluorescent probes for applications in molecular biology.

## Patentansprüche

1. Naphthalimidofurane der allgemeinen Formel I: 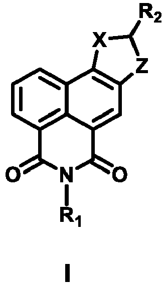
worin R₁ für H, C1 bis C12-Alkyl, Allyl, Propargyl, Benzyl, Glykolketten mit 1 bis 8 Glykol-(-OCH₂CH₂-)-wiederholenden, mit einem C1 bis C12-O-Alkyl Substituenten beendeten, Einheiten, oder für eine OH-Gruppe steht,
wobei R₂ für COOR₁, CON(R₁)₂, worin die beiden R₁ Substituenten gleich oder verschieden sein können, CONH(CH₂)ₓN(R₁)₂, worin x 2 bis 6 ist und worin die beiden R1 Substituenten gleich oder verschieden sein können, oder für CONH(CH₂)ₓNY, worin x wie oben definiert ist und Y für -CH₂-A-CH₂CH₂- steht, worin A CH₂, CH₂CH₂, CH₂O, CH₂S, CH₂SO, CH₂SO₂, CH₂NH, CH₂NR₁ ist, steht,
wobei X = O, Z = CH; R₂C und Z mit einer Doppelbindung gebunden sind (R₂C=Z) oder wobei X = CH, Z = O; R₂C und X mit einer Doppelbindung gebunden sind (R₂C=X).

2. Verwendung der Naphthalimidofurane der allgemeinen Formel I nach Anspruch 1 bei der Herstellung der Fluoreszenzsonden für selektive intrazelluläre Lokalisation.

3. Verwendung der Naphthalimidofurane der allgemeinen Formel I nach Anspruch 1 als Fluoreszenzsonden für Anwendung in der Molekularbiologie.

## Revendications

1. Naphthalimidofuranes de formule générale I: 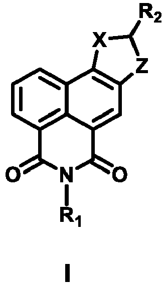
dans laquelle R₁ est H, un alkyle C1 à C12, allyle, propargyle, benzyle, chaînes glycol avec 1 à 8 unités répétées glycol (-OCH₂CH₂-) terminées avec un substituant O-alkyle C1 à C12, ou un groupe OH,
où R₂ est COOR₁, CON(R₁)₂, où les deux substituants R₁ peuvent être identiques ou différents, CONH(CH₂)ₓN(R₁)₂, où x est 2 à 6 et où les deux substituants R1 peuvent être identiques ou différents, CONH(CH₂)ₓNY, où x est comme défini ci-dessus et Y est -CH₂-A-CH₂CH₂-, où A est CH₂, CH₂CH₂, CH₂O, CH₂S, CH₂SO, CH₂SO₂, CH₂NH, CH₂NR₁,
où X = O, Z = CH; R₂C et Z sont liés par une liaison double (R₂C=Z) ou où X = CH, Z = O; R₂C et X sont liés par une liaison double (R₂C=X).

2. Utilisation des naphthalimidofuranes de formule générale I selon la revendication 1 pour préparation des sondes de fluorescence pour la localisation intracellulaire sélective.

3. Utilisation des naphthalimidofuranes de formule générale I selon la revendication 1 en tant que sondes de fluorescence pour les applications en biologie moléculaire.
